# EUROPÄISCHE PATENTANMELDUNG

(11) **EP 2 444 015 A1**
(43) Veröffentlichungstag der Anmeldung: **25.04.2012**
(21) Anmeldenummer: 10188102.7
(22) Anmeldetag: 19.10.2010
(51) Int. Cl.: A61B 17/54, A61B 17/00, A61B 17/32, A61B 19/00

(54) **Vorrichtung zur Behandlung der menschlichen Haut**

(71) Anmelder: La Fontaine, Helmut, 29604 Marbella Málaga (ES)
(72) Erfinder: La Fontaine, Helmut, 29604 Marbella Málaga (ES)
(74) Vertreter: Geitz Truckenmüller Lucht

(57) **Zusammenfassung**

Bekannte Methoden zur Microdermabrasion sehen vor, dass Kristalle als lose Schüttung auf die Haut gestrahlt werden. Der auf diese Weise behandelbare Bereich der Haut ist relativ klein und die gleichzeitige Absaugung des aufgestrahlten Materials ist aufwändig und sensibel. Die Erfindung soll eine Lösung angeben, mit der eine großflächigere Behandlung einfach, funktionssicher und für den Patienten risikofrei erfolgen kann.
Dies gelingt durch den Einsatz von Abrasionstabletten (21), die als Abrasionsmittel Kristalle und/oder kosmetische Wirkstoffpulver enthalten. Durch die bei der Reibung der Abrasionstablette auf der Haut entstehende Reibungswärme lösen sich die Abrasionsmittel, wirken auf der Haut und können leicht wieder abgesaugt werden. Eine gleichzeitige Behandlung mit Kristallen und kosmetischen Wirkstoffen verbessert das Ergebnis zusätzlich.

## Beschreibung

Die vorliegende Erfindung betrifft eine Vorrichtung zur Behandlung der menschlichen Haut, insbesondere mittels Microdermabrasion, mit einem Griffgehäuse und einer Abrasionseinheit zur Kontaktierung der Haut im Rahmen der Behandlung, wobei das Griffgehäuse einen Antrieb zur Übertragung einer Drehbewegung auf die Abrasionseinheit aufweist.

Eine derartige Abrasionsvorrichtung, jedoch zur Durchführung einer Dermabrasion, ist bereits aus der europäischen Patentanmeldung EP 2 082 696 A1 bekannt. Eine derartige Abrasionsvorrichtung sieht eine Abrasionseinheit vor, welche mithilfe eines Antriebs in Drehbewegungen versetzt werden kann. Auf der Behandlungsseite der Abrasionseinheit ist hierbei eine Abrasionsfläche vorgesehen, welche Erhebungen aufweist, die für eine Abtragung der obersten Hautschichten bei der Anwendung auf der menschlichen Haut sorgen.

Grundsätzlich handelt es sich bei der Dermabrasion um eine mechanische Abtragung der obersten Schicht der Epidermis der menschlichen Haut. Hierzu sind eine Reihe verschiedener Methoden bekannt, wie beispielsweise der sich drehende Körper mit einer speziellen Oberflächenrauhigkeit gemäß der vorstehend genannten Patentanmeldung. Das ebenfalls bekannte Verfahren "Diamond Peel" verwendet hierzu insbesondere einen Körper mit einer Oberfläche aus Diamantkristallen. Die genannten Behandlungsmethoden werden eingesetzt zur Bekämpfung von Faltenbildung, Pigmentstörungen, Narbenbildung, bei verhornten Hautstellen und abgestorbenen Hautzellen. Ferner handelt es sich bei der Dermabrasionstechnik um eine professionelle Gesichtsbehandlung, die unter anderem eine Erneuerung und Verjüngung des Hautreliefs bewirken soll.

Die häufigste Anwendung der Dermabrasion ist die sogenannte Microdermabrasion, bei welcher Kristalle, die als lose Schüttung vorliegen, auf die Haut gestrahlt werden. Beim Auftreffen der Kristalle auf der Haut werden die abgestorbenen und/oder verhornten Hautzellen der Epidermis abgelöst und die Kristalle zusammen mit den abgelösten Hautzellen über eine Absaugvorrichtung wieder entfernt. Da es erforderlich ist, die Kristalle mit einem gewissen Druck auf die Haut aufzustrahlen, ist der Aktionsbereich bei den diese Technik umsetzenden Geräten relativ klein und die Technik zum Strahlen und gleichzeitigen Absaugen aufwändig und fehleranfällig.

Vor dem Hintergrund dieses Standes der Technik liegt der vorliegenden Erfindung die Aufgabe zugrunde, eine Microdermabrasion zu bewerkstelligen, bei welcher der Aktionsradius vergrößert werden kann und welche die Problematik des gleichzeitigen Aufstrahlens und wieder Absaugens der verwendeten Kristalle behebt.

Dies gelingt durch eine Vorrichtung zur Behandlung der menschlichen Haut, gemäß den Merkmalen des Anspruchs 1. Weitere sinnvolle Ausgestaltungen dieser Vorrichtung sind den Unteransprüchen zu entnehmen.

Erfindungsgemäß verwendet eine derartige Vorrichtung eine Abrasionseinheit, welche aus wenigstens einer Abrasionstablette besteht, welche in einem Träger aufgenommen ist.

Der Träger weist hierzu eine Aufnahme auf, in welche die Abrasionstablette entweder eingesetzt werden kann oder mit der er fest verbunden ist. Der Träger selbst wird durch einen Antrieb, welcher in einem Griffgehäuse der Vorrichtung aufgenommen ist, in Rotation versetzt, während die Abrasionstablette in Kontakt mit der zu behandelnden Haut gebracht wird. Aufgrund der Drehbewegung, welche die Abrasionstablette in Kontakt mit der Haut ausführt, entsteht eine Reibung, wobei aufgrund der Reibungswärme das in der Abrasionstablette enthaltene Bindemittel gelöst wird. Hierdurch treten einzelne Bestandteile der Abrasionstablette aus und geraten zwischen die behandelte Haut und die sich drehende Abrasionstablette, so dass die austretenden Bestandteile als Abrasionsmittel wirken können.

Diese Abrasionsmittel sind gemeinsam mit den Bindemitteln zu der Abrasionstablette zusammengefügt, wobei die Abrasionstablette bevorzugt durch Pressen hergestellt wird.

Neben den an sich bekannten und hier nicht näher ausgeführten Bindemitteln können als Abrasionsmittel in der Abrasionstablette insbesondere Kristalle verwendet werden, welche entsprechend der bekannten Verfahren zur Microdermabrasion die zu behandelnden Hautschichten von abgestorbenen bzw. verhornten Hautzellen befreien. Ein besonders günstiges Abriebsergebnis wird erzielt, sofern diese Kristalle in unterschiedlichen Korngrößen vorliegen, so dass unterschiedliche Rauhigkeiten der Oberfläche erzielt und somit ein unterschiedlicher Abrieb an der Hautoberfläche erreicht wird. Ebenfalls ist es möglich, unterschiedliche Abrasionstabletten mit Kristallen unterschiedlicher Körnung vorzusehen, um Tabletten verschiedener Rauhigkeitsgrade anbieten zu können.

Als zu verwendende Kristalle wurden insbesondere mit Aluminiumoxidkristallen (Al₂O₃) besonders gute Ergebnisse erzielt. Eine Verwendung anderer Kristalle ist jedoch ebenfalls möglich. Ergänzend zu den Kristallen können auch kosmetische Wirkstoffpulver in der Abrasionstablette vorhanden sein, so dass während der Abrasion zugleich eine kosmetische Behandlung der Haut vorgenommen werden kann, und dadurch ein zweiter Behandlungsdurchgang entfallen kann. In dem Fall, dass eine Behandlung in zwei getrennten Durchgängen gewünscht ist, kann ohne weiteres auch eine Abrasionstablette lediglich mit den kosmetischen Wirkstoffpulvern, welche durch die oben genannten Bindemittel zusammengehalten werden, eingesetzt werden. Die genannten kosmetischen Wirkstoffpulver können unterschiedliche Wirkungen auf die Haut erzielen, so beispielsweise eine beruhigende, anregende, feuchtigkeitsspendende oder auch regenerierende Wirkung.

Durch den Einsatz einer Abrasionstablette ist zwar sichergestellt, dass die Abrasionsmittel, sei es in Form von Kristallen oder auch in Form der kosmetischen Wirkstoffpulver nicht auf die Haut aufgestrahlt werden müssen, dennoch ist es mitunter gewünscht, eine Absaugung der aufgebrachten Abrasionsmittel bzw. auch der Bindemittel zu gewährleisten. Soweit eine derartige Absaugung nicht besteht, wird der beim Behandeln entstehende Abrieb im Verbund mit den Hautpartikeln nach der Behandlung abgenommen. Eine Absaugung wird dadurch realisiert, dass das Griffgehäuse, an welchem das Bedienungspersonal die Vorrichtung halten kann, einen rohrförmigen Behandlungsabschnitt aufweist, in welchen die Abrasionseinheit eingesetzt ist. In diesem Behandlungsabschnitt ist die Abrasionseinheit so angeordnet, dass die Abrasionstablette zum Kontaktieren der Haut über eine Vorderkante des rohrförmigen Behandlungsabschnitts hervorragt und mit dem hervorragenden Teil mit der Haut in Berührung kommen kann. Rings um die Abrasionseinheit ist ein Luftspalt freigelassen, über welchen eine an den rohrförmigen Behandlungsabschnitt angeschlossene Absaugvorrichtung einen Saugdruck im Behandlungsabschnitt aufbauen kann. Abgetragene Hautpartikel sowie die Abrasionsmittel und das gelöste Bindemittel werden über diesen Saugdruck an der Abrasionseinheit vorbei in Richtung der Absaugvorrichtung befördert.

Die gleiche Konfiguration trifft auch auf eine Vorrichtung ohne Absaugung zu, wobei in diesem Fall die Absaugvorrichtung selbstverständlich entfällt.

Die Abrasionseinheit kann gegenüber dem Griffgehäuse derart federnd gelagert sein, dass bei einer unbelasteten Abrasionseinheit diese über die Vorderkante des Behandlungsabschnitts herausragt. Im Falle einer Druckbeaufschlagung der Abrasionseinheit, etwa beim Aufsetzen auf die Haut des Patienten, wirkt dann die Federung der Abrasionseinheit, welche im Bereich des Trägers angeordnet ist. Hierdurch kann die Abrasionseinheit in Richtung des Inneren des Behandlungsabschnitts ausweichen, so dass bei einem zu starken Druck der Vorrichtung auf die menschliche Haut, welcher ansonsten für eine Verletzung sorgen würde, ein Einrücken der Abrasionseinheit bewirkt. Mit Vorteil kann die Abrasionseinheit so weit einrücken, dass diese hinter der Vorderkante des Behandlungsabschnittes liegt, so dass an dieser Stelle nur noch ein höchster zulässiger Druck auf die Haut wirkt. Dieser ist so einzustellen, dass bei diesem höchsten Druck die Haut nicht verletzt werden kann.

Wie bereits dargelegt, kann die Abrasionstablette mit dem Träger fest verbunden sein. In diesem Fall handelt es sich um eine Einweg-Lösung, bei welcher die Abrasionstablette lediglich für eine Behandlung benutzt werden kann und dann zusammen mit dem Träger entsorgt wird.

In diesem Fall ist es sinnvoll, die Kupplung zwischen dem Träger und der Antriebsachse des Antriebs so zu gestalten, dass zunächst beim Einsetzen der Abrasionseinheit eine Verbindung mit der Antriebsachse des Antriebs hergestellt wird, jedoch beim Lösen dieser Verbindung die Kupplung des Trägers zerstört wird, so dass sichergestellt ist, dass ein nochmaliges Einsetzen des Einweg-Trägers verhindert ist.

Umgekehrt kann die Abrasionstablette lösbar mit einem Träger verbunden werden, so dass der Träger wieder verwendbar ist. Insbesondere ist in diesem Fall ein geeignetes, lösbares Haltemittel an der Abrasionstablette vorzusehen, welches mit einem Gegenhaltemittel des Trägers zusammenwirkt. Der Träger sollte in diesem Fall entsprechend so ausgestaltet sein, dass eine Verbindung des Trägers mit der Antriebsachse des Antriebs derart lösbar ist, dass die Kupplung des Trägers zerstörungsfrei von der Antriebsachse zu lösen ist.

Eine spezielle Ausgestaltung der Abrasionstablette sieht vor, dass diese einen zentralen Absaugkanal aufweist, über welchen durch die Abrasionstablette hindurch eine Absaugung des Abriebs der Abrasionstablette und der Haut erfolgen kann. In diesem Falle weist der Träger ebenfalls einen entsprechenden Absaugkanal auf, welcher sich, gegebenenfalls unter Zwischenlage von Verbindungsmitteln, wie etwa Schläuchen oder Röhren, hin zu einer Absaugvorrichtung erstreckt. Diese Absaugvorrichtung ihrerseits baut wiederum in dem Absaugkanal einen Saugdruck auf, mit dessen Hilfe der Abrieb von der Absaugvorrichtung angesaugt und damit aus dem Eingriff der Abrasionstablette bzw. der Haut verbracht werden kann.

Bei der Gestaltung der Abrasionstabletten bestehen einige Freiheiten, sowohl was die Form, als auch die Farbe angeht. Insbesondere sollte aufgrund der rotatorischen Bewegungen die Tablette eine rotationssymmetrische Gestaltung aufweisen, also insbesondere rund, quadratisch oder sechseckig sein, wobei auch beliebige andere mehreckige Formen möglich sind. Auch unsymmetrische Formen sollen von der Erfindung erfasst sein, selbst wenn ihr Schwerpunkt außerhalb der Drehachse gelagert ist. Aufgrund der möglichen, verschiedenen Formen und Farben fällt es leicht, Abrasionstabletten bestimmten Behandlungsbildern und -zielen zuzueignen, da eine Unterscheidung verschiedener Abrasionstabletten aufgrund dieser Merkmale ohne weiteres möglich ist.

Um den Aktionsradius der Vorrichtung weiter zu vergrößern, kann ein Träger auch dazu hergerichtet sein, mehrere Abrasionstabletten auf einmal zu tragen, wobei bevorzugter Maßen eine wiederum rotationssymmetrische Anordnung zu wählen ist, um eine gleichmäßige Beaufschlagung der behandelten Haut zu gewährleisten.

Der Antrieb kann sämtliche bekannte Antriebsarten umfassen, insbesondere ist er jedoch bevorzugter Maßen als elektrischer oder pneumatischer Antrieb ausgelegt. Der Antrieb erzeugt insbesondere eine rotierende oder eine oszillierende Drehbewegung, wodurch wiederum die Art der Behandlung beeinflusst werden kann. Auch eine Abfolge von oszillierenden und rotierenden Drehbewegungen kann realisiert werden.

Die vorstehend beschriebene Erfindung wird im folgenden anhand eines Ausführungsbeispiels näher erläutert.

Es zeigen:
- Fig. 1:: eine Vorrichtung zum Behandeln der menschlichen Haut in einer seitlichen Querschnittsdarstellung,
- Fig. 2:: eine Abrasionseinheit bestehend aus einer Abrasionstablette und einem Träger, in einer perspektivischen Darstellung von schräg oben
- Fig. 3:: die Abrasionseinheit gemäß Fig. 2 in einer Explosionsdarstellung von schräg oben, sowie
- Fig. 4:: drei Abrasionseinheiten mit unterschiedlichen Anzahlen an Abrasionstabletten in perspektivischer Darstellung von schräg oben.

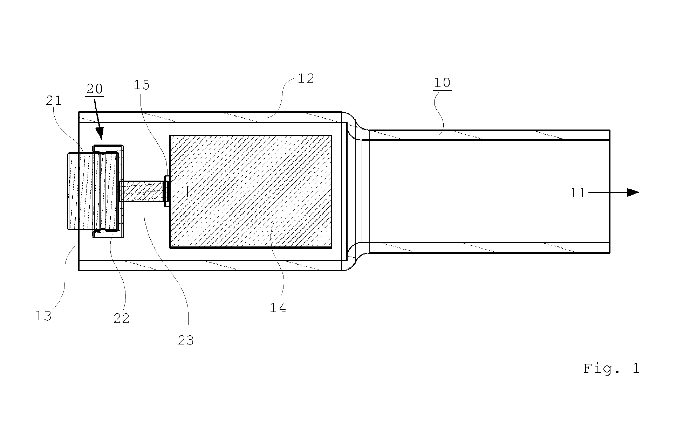

Fig. 1 zeigt eine Vorrichtung zur Behandlung der menschlichen Haut, welche im wesentlichen nach außen hin durch ihr Griffgehäuse 10 bestimmt ist. In dem Griffgehäuse 10 ist ein Antrieb 14 aufgenommen, welcher eine Abrasionseinheit 20 in Drehbewegung versetzt. Die Abrasionseinheit 20 besteht hierbei aus einer Abrasionstablette 21 und einem Träger 22, wobei letztere über eine Federung 23 und eine Verbindung 15 mit der Antriebsachse des Antriebs 14 verbunden ist. Der Antrieb 14 bewirkt eine bedarfsweise rotierende oder oszillierende Drehbewegung der Abrasionseinheit 20.

Die Abrasionstablette 21 besteht aus einer Mischung von Aluminiumoxidkristallen in verschiedenen Kristallgrößen, welche zusammen mit pulverförmigen Kosmetikwirkstoffen und Bindemitteln zu einer Abrasionstablette verpresst sind. Die Vorrichtung wird mit dem Griffgehäuse 10 derart auf die menschliche Haut aufgesetzt, dass diese in Kontakt mit der Oberfläche der Abrasionstablette 21 gerät. Durch die mithilfe des Antriebs 14 realisierte Drehbewegung entsteht eine Reibung der Abrasionstablette 21 auf der menschlichen Haut, wobei durch die hierdurch entstehende Reibungswärme die Bindemittel gelöst und damit die Abrasionsmittel in Form der Aluminiumoxidkristalle und der kosmetischen Wirkstoffpulver freigesetzt werden. Letztere treten nunmehr in Wechselwirkung mit der Haut, wobei die Kristalle eine Abtragung der abgestorbenen und der verhornten Hautschichten bewirken, während die kosmetischen Wirkstoffpulver in die Haut einmassiert werden.

Bei einem zu starken Druck der Abrasionstablette 21 auf die zu behandelnde Haut weicht die Abrasionseinheit 20 aufgrund der Federung 23 in das Griffgehäuse 10, insbesondere einen Behandlungsabschnitt 12 zurück, nämlich soweit, dass im Extremfall die Abrasionstablette 21 bis hinter eine Vorderkante 13 des Behandlungsabschnitts 12 einrücken kann. Hierdurch wird verhindert, dass durch einen zu starken Druck auf die menschliche Haut eine zu starke Abrasion bewirkt wird und es zu Verletzungen kommen kann.

Der entstehende Abrieb im Bereich der zu behandelnden Haut kann schließlich über eine Absaugvorrichtung entfernt werden, welche mit einem Absaugschlauch 11 verbunden ist. Dieser ist seinerseits mit dem Griffgehäuse 10 derart verbunden, dass in dem Behandlungsabschnitt 12 ein Saugdruck angelegt wird. Der um die Abrasionstablette 21 herum entstehende Abrieb wird aufgrund dieses Saugdrucks in das Innere des Behandlungsabschnitts 12 eingesaugt und vorbei an dem Antrieb 14 in Richtung des Absaugschlauchs 11 entfernt.

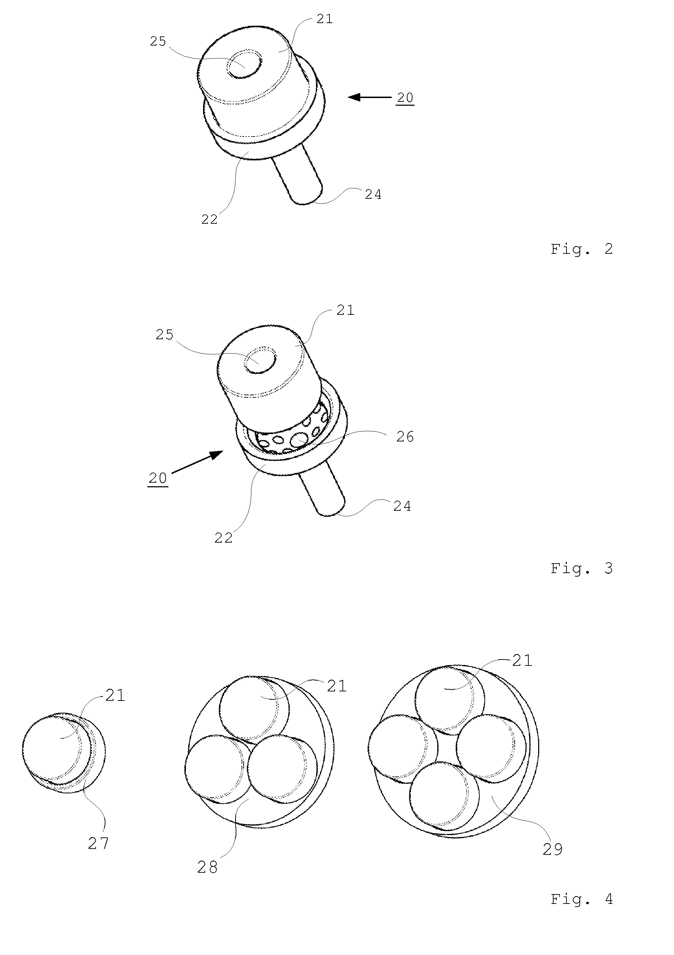

Fig. 2 zeigt eine Abrasionseinheit 20, welche aus einer Abrasionstablette 21 und einem Träger 22 besteht. Die Abrasionstablette 21 kann hierbei entweder direkt auf dem Träger 22 unlösbar befestigt sein, so dass es sich um eine Einweg-Lösung handelt. In diesem Fall ist eine an der Achse des Trägers 22 angebrachte Kupplung 24 derart ausgelegt, dass diese nach dem Eingriff in die Antriebsachse des Antriebs 14 nicht zerstörungsfrei wieder von dieser gelöst werden kann. Aufgrund des Kupplungsbruchs kann die Einweg-lösung nicht nochmals verwendet werden, so dass auf diese Weise sichergestellt ist, dass eine zweite Behandlung mit derselben Abrasionseinheit 20 bei einem anderen Probanden nicht erfolgen kann. Benutzte Abrasionseinheiten 20 sind damit klar erkennbar.

Eine zweite Möglichkeit besteht jedoch darin, dass die Träger 22 wieder verwendbar ausgestaltet sind. In diesem Fall ist eine Kupplung 24 derart ausgestaltet, dass diese nach einer Verbindung mit der Antriebsachse des Antriebs 14 wieder zerstörungsfrei gelöst werden kann, beispielsweise durch Verschraubung, Verclipsung oder dergleichen mehr. Die Abrasionstablette 21 ist in diesem Fall unter Zwischenlage von Haltemitteln, welche in Gegenhaltemittel des Trägers eingreifen, mit diesem verbunden und ebenfalls austauschbar, so dass mehrere verschiedene Abrasionstabletten 21 eingesetzt werden können. Die Abrasionstabletten 21 können unterschiedliche Formen aufweisen, wobei es sich üblicherweise um rotationssymmetrische Formen handeln wird, welche bei einer Rotation eine gleichmäßige Beaufschlagung der Haut mit sich bringen. Die vorliegend gezeigte Abrasionstablette 21 weist einen Absaugkanal 25 auf, über welchen der im Rahmen der Behandlung entstehende Abrieb zentral abgesaugt werden kann. Der Absaugkanal 25 setzt sich in diesem Fall durch den Träger 22 hindurch fort und erstreckt sich, insbesondere unter Zwischenlage von Verbindungsmitteln, bis hin zu einer Absaugvorrichtung, welche einen Saugdruck innerhalb des Absaugkanals erzeugt.

Fig. 3 zeigt die Variante einer lösbaren Abrasionstablette 21, unterhalb derer bei dem gezeigten Träger 22 ein Absaugeinsatz 26 vorgesehen ist. Dieser weist Öffnungen auf, über welche der durch den Absaugkanal 25 der Abrasionstablette 21 abgesaugte Abrieb in Richtung einer Absaugvorrichtung befördert werden kann.

Wie bereits dargelegt, können die Abrasionstabletten 21 unterschiedliche Formen annehmen. Hierbei ist daran gedacht, dass aufgrund verschiedener Form- und Farbgebung der Abrasionstabletten diese vom Behandlungspersonal leicht unterscheiden werden können, so dass es möglich ist, für bestimmte Behandlungen ein bestimmtes Farb- oder Formschema vorzugeben.

Fig. 4 zeigt eine weitere Variante der Erfindung, nämlich die Möglichkeit, mehrere Abrasionstabletten 21 auf einen Träger 22 unterzubringen. Hierzu ist in der linken Darstellung zunächst ein Einzelträger 27 gezeigt, welcher eine einzelne Abrasionstablette 21 trägt. Durch eine Vergrößerung des Trägers wird ein Dreifachträger 28 geschaffen, auf welchem insgesamt drei Abrasionstabletten 21 Platz finden. Durch eine weitere Erweiterung können auch vier oder mehr Abrasionstabletten 21 auf einem Vierfachträger 29 oder höhergradigen Mehrfachträgern angeordnet werden. Je nachdem, welcher Hautbereich des Körpers behandelt werden soll, kann hierdurch die Flächengröße der Behandlung angepasst werden, ohne hierbei die Dauer der Behandlung zu sehr zu vergrößern.

Vorstehend beschrieben ist somit eine Vorrichtung zur Behandlung der menschlichen Haut, insbesondere mittels Microdermabrasion, welche unter Verwendung einer Abrasionstablette, in welcher die Abrasionsmittel zusammen mit Bindemitteln gebunden sind, das Aufbringen der Abrasionsmittel auf die menschliche Haut vereinfacht. Auf ein Aufstrahlen der Abrasionsmittel auf die menschliche Haut kann dadurch verzichtet werden, während gleichzeitig der Aktionsradius der Anordnung vergrößert wird. Durch eine gegebenenfalls überlagerte Absaugung kann letztlich der gleiche Effekt wie bei herkömmlichen Microdermabrasionsverfahren erzielt werden, welcher jedoch gleichzeitig über eine größere Fläche wirkt und eine gleichzeitige Einmassierung von kosmetischen Wirkstoffpulvern in die zu behandelnde Haut ermöglicht.

### BEZUGSZEICHENLISTE

- 10: Griffgehäuse
- 11: Absaugschlauch
- 12: Behandlungsabschnitt
- 13: Vorderkante
- 14: Antrieb
- 15: Verbindung
- 20: Abrasionseinheit
- 21: Abrasionstablette
- 22: Träger
- 23: Federung
- 24: Kupplung
- 25: Absaugkanal
- 26: Absaugeinsatz
- 27: Einzelträger
- 28: Dreifachträger
- 29: Vierfachträger

## Patentansprüche

1. Vorrichtung zur Behandlung der menschlichen Haut, insbesondere mittels Microdermabrasion, mit einem Griffgehäuse (10) und einer Abrasionseinheit (20) zur Kontaktierung der Haut im Rahmen der Behandlung, wobei das Griffgehäuse (10) einen Antrieb (14) zur Übertragung einer Drehbewegung auf die Abrasionseinheit (20) aufweist,
**dadurch gekennzeichnet, dass** die Abrasionseinheit (20) wenigstens eine Abrasionstablette (21) und einen diese aufnehmenden Träger (22) umfasst, wobei die Abrasionstablette (21) aus einer Mischung von Abrasions- und Bindemitteln, vorzugsweise durch Pressen, hergestellt ist.

2. Vorrichtung gemäß Anspruch 1, **dadurch gekennzeichnet, dass** die Abrasionstablette (21) als Abrasionsmittel Kristalle, vorzugsweise mehrerer unterschiedlicher Korngrößen, und/oder kosmetische Wirkstoffpulver enthält.

3. Vorrichtung gemäß einem der Ansprüche 1 oder 2, **dadurch gekennzeichnet, dass** das Griffgehäuse (10) einen rohrförmigen Behandlungsabschnitt (12) aufweist, in welchem die Abrasionseinheit (20) derart endständig aufgenommen ist, dass zumindest die Abrasionstablette (21) wenigstens teilweise über eine Vorderkante (13) des Behandlungsabschnitts (12) hinaus aus diesem hervorragt.

4. Vorrichtung gemäß Anspruch 3, **dadurch gekennzeichnet, dass** die Abrasionseinheit (20) gegenüber dem Griffgehäuse (10) derart federnd gelagert ist, dass die Abrasionseinheit (20) bei entsprechendem Druck bis hinter die Vorderkante (13) des Griffgehäuses einrücken kann.

5. Vorrichtung gemäß einem der Ansprüche 3 oder 4, **dadurch gekennzeichnet, dass** der Behandlungsabschnitt (12), vorzugsweise über einen Absaugschlauch (11), mit einer Absaugvorrichtung zur Erzeugung eines Saugdrucks innerhalb des Behandlungsabschnitts (12) verbunden ist.

6. Vorrichtung gemäß einem der vorhergehenden Ansprüche,
**dadurch gekennzeichnet, dass** die Abrasionstablette (21) mit dem Träger (22) fest verbunden ist.

7. Vorrichtung gemäß Anspruch 6, **dadurch gekennzeichnet, dass** eine Verbindung (15) zwischen dem Träger (22) und dem Antrieb (14) entlang einer Antriebsachse über eine in eine Aufnahme des Antriebs (14) eingreifende Kupplung (24) des Trägers (22) realisiert ist, wobei diese Verbindung (15) nur unter Zerstörung der Kupplung (24) des Trägers (22) lösbar ist.

8. Vorrichtung gemäß einem der Ansprüche 1 bis 5, **dadurch gekennzeichnet, dass** die Abrasionstablette (21), vorzugsweise unter Zwischenlage in Gegenhaltemittel des Trägers (22) eingreifender Haltemittel, mit diesem austauschbar verbunden ist.

9. Vorrichtung gemäß Anspruch 8, **dadurch gekennzeichnet, dass** eine Verbindung (15) zwischen dem Träger (22) und dem Antrieb (14) entlang einer Antriebsachse über eine in eine Aufnahme des Antriebs (14) eingreifende Kupplung (24) des Trägers (22) realisiert ist, wobei diese Verbindung (15) zerstörungsfrei lösbar ist.

10. Vorrichtung gemäß einem der vorhergehenden Ansprüche, **dadurch gekennzeichnet, dass** die Abrasionstablette (21) einen Absaugkanal (25) aufweist, welcher sich durch den Träger (22) hindurch, bedarfsweise unter Zwischenlage von Verbindungsmitteln, zu einer einen Saugdruck in dem Absaugkanal (25) erzeugenden Absaugvorrichtung erstreckt.

11. Vorrichtung gemäß einem der vorhergehenden Ansprüche, **dadurch gekennzeichnet, dass** die Abrasionstablette (21) rotationssymmetrisch ist, vorzugsweise rund, quadratisch oder sechseckig.

12. Vorrichtung gemäß einem der vorhergehenden Ansprüche, **dadurch gekennzeichnet, dass** der Träger (22) mehrere Abrasionstabletten (21) aufweist.

13. Vorrichtung gemäß einem der vorhergehenden Ansprüche, **dadurch gekennzeichnet, dass** es sich bei dem Antrieb (14) um einen elektrischen oder pneumatischen Antrieb handelt, welcher eine rotierende oder eine oszillierende Drehbewegung erzeugt.

## Geänderte Patentansprüche

### Geänderte Patentansprüche gemäss Regel 137(2) EPÜ.

**1.** Vorrichtung zur Behandlung der menschlichen Haut, insbesondere mittels Microdermabrasion, mit einem Griffgehäuse (10) und einer Abrasionseinheit (20) zur Kontaktierung der Haut im Rahmen der Behandlung, wobei das Griffgehäuse (10) einen Antrieb (14) zur Übertragung einer Drehbewegung auf die Abrasionseinheit (20) aufweist, wobei die Abrasionseinheit (20) wenigstens eine Abrasionstablette (21) und einen diese aufnehmenden Träger (22) umfasst,
**dadurch gekennzeichnet, dass** die Abrasionstablette (21) aus einer Mischung von Abrasions- und Bindemitteln durch Pressen hergestellt ist.

**2.** Vorrichtung gemäß Anspruch 1, **dadurch gekennzeichnet, dass** die Abrasionstablette (21) als Abrasionsmittel Kristalle, vorzugsweise mehrerer unterschiedlicher Korngrößen, und/oder kosmetische Wirkstoffpulver enthält.

**3.** Vorrichtung gemäß einem der Ansprüche 1 oder 2, **dadurch gekennzeichnet, dass** das Griffgehäuse (10) einen rohrförmigen Behandlungsabschnitt (12) aufweist, in welchem die Abrasionseinheit (20) derart endständig aufgenommen ist, dass zumindest die Abrasionstablette (21) wenigstens teilweise über eine Vorderkante (13) des Behandlungsabschnitts (12) hinaus aus diesem hervorragt.

**4.** Vorrichtung gemäß Anspruch 3, **dadurch gekennzeichnet, dass** die Abrasionseinheit (20) gegenüber dem Griffgehäuse (10) derart federnd gelagert ist, dass die Abrasionseinheit (20) bei entsprechendem Druck bis hinter die Vorderkante (13) des Griffgehäuses einrücken kann.

**5.** Vorrichtung gemäß einem der Ansprüche 3 oder 4, **dadurch gekennzeichnet, dass** der Behandlungsabschnitt (12), vorzugsweise über einen Absaugschlauch (11), mit einer Absaugvorrichtung zur Erzeugung eines Saugdrucks innerhalb des Behandlungsabschnitts (12) verbunden ist.

**6.** Vorrichtung gemäß einem der vorhergehenden Ansprüche,
**dadurch gekennzeichnet, dass** die Abrasionstablette (21) mit dem Träger (22) fest verbunden ist.

**7.** Vorrichtung gemäß Anspruch 6, **dadurch gekennzeichnet, dass** eine Verbindung (15) zwischen dem Träger (22) und dem Antrieb (14) entlang einer Antriebsachse über eine in eine Aufnahme des Antriebs (14) eingreifende Kupplung (24) des Trägers (22) realisiert ist, wobei diese Verbindung (15) nur unter Zerstörung der Kupplung (24) des Trägers (22) lösbar ist.

**8.** Vorrichtung gemäß einem der Ansprüche 1 bis 5, **dadurch gekennzeichnet, dass** die Abrasionstablette (21), vorzugsweise unter Zwischenlage in Gegenhaltemittel des Trägers (22) eingreifender Haltemittel, mit diesem austauschbar verbunden ist.

**9.** Vorrichtung gemäß Anspruch 8, **dadurch gekennzeichnet, dass** eine Verbindung (15) zwischen dem Träger (22) und dem Antrieb (14) entlang einer Antriebsachse über eine in eine Aufnahme des Antriebs (14) eingreifende Kupplung (24) des Trägers (22) realisiert ist, wobei diese Verbindung (15) zerstörungsfrei lösbar ist.

**10.** Vorrichtung gemäß einem der vorhergehenden Ansprüche, **dadurch gekennzeichnet, dass** die Abrasionstablette (21) einen Absaugkanal (25) aufweist, welcher sich durch den Träger (22) hindurch, bedarfsweise unter Zwischenlage von Verbindungsmitteln, zu einer einen Saugdruck in dem Absaugkanal (25) erzeugenden Absaugvorrichtung erstreckt.

**11.** Vorrichtung gemäß einem der vorhergehenden Ansprüche, **dadurch gekennzeichnet, dass** die Abrasionstablette (21) rotationssymmetrisch ist, vorzugsweise rund, quadratisch oder sechseckig.

**12.** Vorrichtung gemäß einem der vorhergehenden Ansprüche, **dadurch gekennzeichnet, dass** der Träger (22) mehrere Abrasionstabletten (21) aufweist.

**13.** Vorrichtung gemäß einem der vorhergehenden Ansprüche, **dadurch gekennzeichnet, dass** es sich bei dem Antrieb (14) um einen elektrischen oder pneumatischen Antrieb handelt, welcher eine rotierende oder eine oszillierende Drehbewegung erzeugt.

## Geänderte Patentansprüche

### Geänderte Patentansprüche gemäss Regel 137(2) EPÜ.

**1.** Vorrichtung zur Behandlung der menschlichen Haut, insbesondere mittels Microdermabrasion, mit einem Griffgehäuse (10) und einer Abrasionseinheit (20) zur Kontaktierung der Haut im Rahmen der Behandlung, wobei das Griffgehäuse (10) einen Antrieb (14) zur Übertragung einer Drehbewegung auf die Abrasionseinheit (20) aufweist, wobei die Abrasionseinheit (20) wenigstens eine Abrasionstablette (21) und einen diese aufnehmenden Träger (22) umfasst,
**dadurch gekennzeichnet, dass** die Abrasionstablette (21) aus einer Mischung aus Aluminiumoxidkristallen, pulverförmigen Kosmetikwirkstoffen und Bindemitteln durch Pressen hergestellt ist.

**2.** Vorrichtung gemäß Anspruch 1, **dadurch gekennzeichnet, dass** das Griffgehäuse (10) einen rohrförmigen Behandlungsabschnitt (12) aufweist, in welchem die Abrasionseinheit (20) derart endständig aufgenommen ist, dass zumindest die Abrasionstablette (21) wenigstens teilweise über eine Vorderkante (13) des Behandlungsabschnitts (12) hinaus aus diesem hervorragt.

**3.** Vorrichtung gemäß Anspruch 2, **dadurch gekennzeichnet, dass** die Abrasionseinheit (20) gegenüber dem Griffgehäuse (10) derart federnd gelagert ist, dass die Abrasionseinheit (20) bei entsprechendem Druck bis hinter die Vorderkante (13) des Griffgehäuses einrücken kann.

**4.** Vorrichtung gemäß einem der Ansprüche 2 oder 3, **dadurch gekennzeichnet, dass** der Behandlungsabschnitt (12), vorzugsweise über einen Absaugschlauch (11), mit einer Absaugvorrichtung zur Erzeugung eines Saugdrucks innerhalb des Behandlungsabschnitts (12) verbunden ist.

**5.** Vorrichtung gemäß einem der vorhergehenden Ansprüche,
**dadurch gekennzeichnet, dass** die Abrasionstablette (21) mit dem Träger (22) fest verbunden ist.

**6.** Vorrichtung gemäß Anspruch 5, **dadurch gekennzeichnet, dass** eine Verbindung (15) zwischen dem Träger (22) und dem Antrieb (14) entlang einer Antriebsachse über eine in eine Aufnahme des Antriebs (14) eingreifende Kupplung (24) des Trägers (22) realisiert ist, wobei diese Verbindung (15) nur unter Zerstörung der Kupplung (24) des Trägers (22) lösbar ist.

**7.** Vorrichtung gemäß einem der Ansprüche 1 bis 4, **dadurch gekennzeichnet, dass** die Abrasionstablette (21), vorzugsweise unter Zwischenlage in Gegenhaltemittel des Trägers (22) eingreifender Haltemittel, mit diesem austauschbar verbunden ist.

**8.** Vorrichtung gemäß Anspruch 7, **dadurch gekennzeichnet, dass** eine Verbindung (15) zwischen dem Träger (22) und dem Antrieb (14) entlang einer Antriebsachse über eine in eine Aufnahme des Antriebs (14) eingreifende Kupplung (24) des Trägers (22) realisiert ist, wobei diese Verbindung (15) zerstörungsfrei lösbar ist.

**9.** Vorrichtung gemäß einem der vorhergehenden Ansprüche, **dadurch gekennzeichnet, dass** die Abrasionstablette (21) einen Absaugkanal (25) aufweist, welcher sich durch den Träger (22) hindurch, bedarfsweise unter Zwischenlage von Verbindungsmitteln, zu einer einen Saugdruck in dem Absaugkanal (25) erzeugenden Absaugvorrichtung erstreckt.

**10.** Vorrichtung gemäß einem der vorhergehenden Ansprüche, **dadurch gekennzeichnet, dass** die Abrasionstablette (21) rotationssymmetrisch ist, vorzugsweise rund, quadratisch oder sechseckig.

**11.** Vorrichtung gemäß einem der vorhergehenden Ansprüche, **dadurch gekennzeichnet, dass** der Träger (22) mehrere Abrasionstabletten (21) aufweist.

**12.** Vorrichtung gemäß einem der vorhergehenden Ansprüche, **dadurch gekennzeichnet, dass** es sich bei dem Antrieb (14) um einen elektrischen oder pneumatischen Antrieb handelt, welcher eine rotierende oder eine oszillierende Drehbewegung erzeugt.
